# EUROPEAN PATENT SPECIFICATION

(11) **EP 1 229 784 B1**
(45) Date of publication and mention of the grant of the patent: **02.02.2005**
(21) Application number: 00981846.9
(22) Date of filing: 16.11.2000
(51) Int. Cl.: A01K 67/027, C12N 5/06

(54) **AN IMPROVED METHOD FOR PRODUCTION OF PORCINE CLONE EMBRYOS VIA SOMATIC CELL NUCLEAR TRANSFER**
VERBESSERTE METHODE ZUR HERSTELLUNG KLONIERTEN SCHWEINEEMBRYOS MITTELS SOMATISCHEN ZELLKERNTRANSFER
PROCEDE AMELIORE POUR LA PRODUCTION D'EMBRYONS DE CLONES PORCINS PAR TRANSFERT NUCLEAIRE DE CELLULES SOMATIQUES

(30) Priority: 19.11.1999 KR 9951551
(43) Date of publication of application: 14.08.2002
(73) Proprietor: Korea Research Institute of Bioscience and Biotechnology, Daejeon 305-333 (KR)
(72) Inventor: HAN, Yong-Mahn, Daejeon 305-345 (KR); LEE, Kyung-Kwang, Daejeon 300-200 (KR); KOO, Deog-Bon, Daejeon 305-345 (KR); PARK, Jung-Sun, Daejeon 305-345 (KR); KANG, Yong-Kook, Daejeon 305-345 (KR); CHOI, Young-Hee, Daejeon 302-792 (KR); YU, Dae-Yeul, Daejeon 305-333 (KR)
(74) Representative: Reinhard - Skuhra - Weise & Partner
(86) International application number: PCT/KR2000/001312
(87) International publication number: WO 2001/035734

(56) References cited:
- WO-A1-97/07668
- WO-A2-98/30683
- WO-A2-99/37143

## Description

### BACKGROUND OF THE INVENTION

### Field of the Invention

The present invention relates to a production method of clone embryos by nuclear transfer, and more particularly, to an improved method for mass production of clone embryos by a novel somatic cell nuclear transfer technique which comprises a fusion between an enucleated recipient oocyte and a somatic cell or a somatic cell nucleus by electric stimulation, an oocyte reconstruction, and an activation of said reconstructed oocytes by secondary electric stimulation.

### Description of the Prior Art

The traditional way of improving traits of a given animal species such as livestock animals had been heavily relied on the hybridization between animals exhibiting superior phenotypes. However, this method has not been considered very advantageous because it generally requires a considerable amount of time to generate genetically superior offspring thus impeding fast regeneration of selected animals. Since the introduction of a new scientific method called nuclear transfer (NT) which utilizes somatic cells of a given animal, however, cloning animals in sheep (Schnieke et al., Science, 278:2130-2133 (1997); Wilmut et al., Nature, 385:810-813 (1997)), goats (Baguisi et al., Nature Biotechnology, 17:456-461 (1999)), mice (Wakayama et al., Nature, 394:369-374 (1998)), cattle (Cibelli et al., Science, 280:1256-1258 (1998); Kato et al., Science, 282:2095-2098 (1998); Wells et al., Biol. Reprod., 60:996-1005 (1999)) and pigs (Onishi et al., Science, 289:1188-1190 (2000); Polejaeva et al., Nature, 407:505-509 (2000)) have been successfully performed. In fact, a variety of somatic cells from cumulus or oviduct (Wakayama et al., Nature, 394:369-374 (1998); Kato et al., Science, 282:2095-2098 (1998)), granulosa (Wells et al., Biol. Reprod., 60:996-1005 (1999)), mammary epithelium (Wilmut et al., Nature, 385:810-813 (1997)), fetal fibroblast (Schnieke et al., Science, 278:2130-2133 (1997); Baguisi et al., Nature Biotechnology, 17:456-461 (1999); Cibelli et al., Science, 280:1256-1258 (1998)) and ear skin cells (Zakhartchenko et al., Mol. Reprod. Dev., 54:264-272 (1999)) have been used as good sources of nuclei donors.

Nevertheless, despite incessant trials, the nuclear transfer technique using somatic cells in pigs had not been successful for a long time (Du et al., Theriogenology, 51:201 (1999); Tao et al., Cloning, 1:55-62 (1999)), and there has been only a line of report on a piglet born from a NT embryo with 4-cell nucleus (Prather et al., Biol. Reprod., 41:414-418 (1989)). Therefore, cloning pigs using somatic cells had been regarded as a very difficult task to accomplish until recent years when a few lines of successful achievements were reported (Onishi et al., Science, 289:1188-1190 (2000); Polejaeva et al., Nature, 407:505-509 (2000)) where the researches were conducted using *in vivo*-derived oocytes as recipient cytoplasts.

lonophore A23187 has been known as a good universal activator for mammalian eggs (Steinhardt et al., Nature, 252:41-43 (1974)) because it can induce cortical granule exocytosis, second polar body extrusion, and pronuclear formation in oocytes of several animal species (Marcus, Mol. Reprod. Dev., 16:1384-1391 (1990); Liu et al., Mol. Reprod. Dev., 49:298-307 (1998); Wang et al., Mol. Reprod. Dev., 51:346-353 (1998). Porcine oocytes treated with the ionophore were also shown to form pronuclei and parthenogenetically develop to blastocyst stage (Funahashi et al., Biol. Reprod., 50:1072-1077 (1994); Wang et al., Biol. Reprod., 60:1020-1028 (1999)).

In general, combined chemical agents such as Ca-ionophore/6-DMAP (Cibelli et al., Science, 280:1256-1258 (1998); De Sousa et al., Cloning, 1:63-69 (1999)), ionomycin/6-DMAP (Wells et al., Biol. Reprod., 60:996-1005 (1999)) or cycloheximide/cytochalasin B (Zakhartchenko et al., J. Reprod. Fertil., 115:325-331 (1999)), have been used to activate bovine oocytes reconstructed with somatic cells. In pigs, some reconstructed eggs activated by exposing them to thimerosal/dithiothreitol were shown to develop into presumptive blastocysts (Tao et al., Cloning, 1:55-62 (1999)).

Electric stimulation has been also known as a potent activator of porcine oocytes (Jolliff and Prather, Biol. Reprod., 56:544-548 (1997); Wang et al., Mol. Reprod. Dev., 51:346-353 (1998)). When porcine NT eggs with fibroblast cells were activated and fused simultaneously by electric stimulation, a few embryos were developed to blastocysts (Du et al., Theriogenology, 51:201 (1999)).

In nuclear transfer using embryonic or somatic cells, it is very common to observe that a portion of the oocyte cytoplasm is removed during enucleation process. The resulting cytoplasm with a reduced volume is then ascribed to subsequent deterioration of the developmental potential as well as the decrease in cell numbers of NT embryos in cattle (Peura et al., Mol. Reprod. Dev., 50:185-191 (1998)). Bovine NT embryos injected with fibroblast cells showed a relatively low developmental rate by showing only 12% of the embryos under the experiment reaching blastocyst stage (Cibelli et al., Science, 280:1256-1258 (1998)), whereas the embryos injected with mural or cumulus granulosa cells resulted in a relatively higher *in vitro* development rate by showing about 50% of the embryos reaching the blastocyst stage (Kato et al., Science, 282:2095-2098 (1998); Wells et al., Biol. Reprod., 60:996-1005 (1999)). The difference in these two different sets of experiments suggests that each different cell type as a nucleus donor may have a different developmental potential, however, the details of the developmental competence of NT porcine embryos *in vitro* have not been clearly answered yet and still need to be further elucidated.

Somatic cell nuclear transfer has become a powerful tool in producing transgenic animals, especially transgenic livestock animals. Cloned transgenic animals have been also produced from transfected fibroblast cells (Schnieke et al., Science, 278:2130-2133 (1997); Cibelli et al., Science, 280:1256-1258 (1998)). However, it should be also noted that genetic modifications on somatic cells is a prerequisite in generating these transgenic pigs by using the nuclear transfer technique mentioned in the above. Despite the recent success achieved in sheep, cattle, mice, goats and pigs using a variety of somatic cell types as nuclear donors, the efficiency of somatic cell nuclear transfer is still very low showing only less than 2%. Therefore, there has been a long-awaited need for the development of a more advanced nuclear transfer technique for more extensive applications to animal industry and biomedical areas.

WO-9830683 discloses a nuclear transfer with differentiated fetal and adult donor cells. The method of nuclear transfer implies the transplantation of donor cell nuclei into enucleated oocytes of the same species as the donor cell.

WO-9937143 describes the full term development of animals from enucleated oocytes reconstituted with adult somatic cell nuclei. An adult somatic cell nucleus is inserted in a recipient enucleated oocyte by microinjection and the oocyte is activated prior to, during, or up to 6 hours after insertion of the nucleus by, inter alia, electroactivation.

WO-9707668 dicloses a method of reconstituting a diploid nucleus into an oocyte which is arrested in the metaphase of the second meiotic division.

### SUMMARY OF THE INVENTION

The object of the present invention is therefore to provide an improved method for mass production of porcine clone embryos via nuclear transfer of somatic cells. As a way to improve the relatively low *in vitro* developmental rates of cloned oocytes generated according to somatic cell nuclear transfer methods, the inventors of the present invention developed a new method of production of porcine clone embryos, wherein matured enucleated porcine oocytes are fused with nuclei derived from cultured somatic cells by electric stimulation, allowed to be remodeled for 1 ∼ 2 h, and then further activated by secondary electric stimulation to increase the viability of said embryos and mediate continuous *in vitro* development. The present invention then will be able to help to set up a concrete way toward the establishment of implantation in cloning pigs.

### BRIEF DESCRIPTION OF THE FIGURES

Figure 1 shows a schematic diagram of the production procedures of porcine clone embryos according to the present invention.
Figure 2 illustrates embodiments of the invention for creating transgenic cell lines and cloned transgenic embryos. Expression of GFP in fibroblast cells (A) and NT blastocyst (C). The same NT embryo (B) was viewed under light microscope.

### DETAILED DESCRIPTION OF THE INVENTION

The present invention relates to an improved method for mass production of porcine clone embryos by somatic cell nuclear transfer comprising a series of steps of (a) enucleation of recipient oocytes; (b) *in vitro* culture of nuclei-donating somatic cells; (c) injection of the somatic cells into the enucleated oocytes; (d) electrofusion of each enucleated oocyte with a donor somatic cell; (e) activation of the reconstructed embryos by secondary electric stimulation; (f) *in vitro* culture of NT embryos and transfer of the NT embryos to recipient pigs.

The present invention can be described in more detail as follows.

First, the present invention is characterized by having an 1∼2 h of oocyte reconstruction period following the oocyte enucleation and the fusion of said oocytes with somatic cells by electric stimulation. In general, it is recommended to use mammalian somatic cells as nuclei-donors except those from humans, and more preferably are fibroblast cells or cumulus cells taken from animals such as pigs, cows, sheep and mice. In the present invention, recipient oocytes were collected from prepubertal porcine ovaries, and immature oocytes were incubated to be matured *in vitro*. Then, only those matured oocytes having first polar bodies were selected and subsequently enucleated to be used. Single donor somatic cells were individually injected into each enucleated oocyte, exposed to primary electric stimulation of 160 V/mm of electric pulse for 30 µsec to induce the fusion between the cytoplasm of each enucleated oocyte and each nucleus of donor somatic cells, respectively. Thus obtained oocytes were then allowed to reconstruct for 1∼2 h by culturing in phosphate buffered saline (PBS) containing fetal bovine serum.

The above reconstructed oocytes are then subject to secondary electric stimulation to be activated, wherein said oocytes are exposed to 120∼150 V/mm of electric pulse for 30 µsec. If the electric stimulation is below 120 V/mm the activation rate becomes low while if it exceeds 150 V/mm the risk for oocyte injury will be increased. These oocytes are then cultured *in vitro* for further development. In the conventional nuclear transfer methods, wherein only one electric stimulation is applied to enucleated oocytes, *in vitro* developmental rate usually results in having less than 5%; in contrast, the present invention by introducing secondary electric stimulation could markedly improve the developmental rate of said oocytes up to 11.6 %, i.e., 130 % increase as compared to those by conventional methods.

The activation of reconstructed oocytes is executed after the transfer of somatic cell nuclei into recipient cytoplasts. Several combined chemical treatments, such as calcium ionophore/6-DMAP or ethanol/cycloheximide/cytochalasin B, have been used to activate the bovine NT oocytes with somatic cells. However, they showed relatively lower rate of activation when porcine NT oocytes were treated with A23187 alone or with the co-agent of A23187/6-DMAP. In general, porcine oocytes showed lower activation rates when treated with a single chemical agent while the porcine oocytes developed into blastocysts when treated with thimerosal/DTT, a combined chemical agent, although their developmental rates were low. In another report, the activation by sulfhydryl reagents showed a low cleavage rate and thus resulting in a subsequently low developmental rate of porcine NT oocytes that could reach the blastocyst. According to the present invention, porcine NT embryos activated by electric stimulation showed a relatively higher developmental rate than those treated with chemical agents.

The developmental abilities of porcine NT eggs induced by different level of electric voltages after electrofusion were examined and the result showed that electric pulse (120V/mm DC pulse) is very effective on activation of porcine NT embryos with somatic cells.

Approximately 70% of reconstructed embryos using pig fibroblasts failed to divide when activated with thimerosal/dithiothreitol treatment (Tao et al., Cloning, 1:55-62 (1999)). In the present invention, the average cleavage rates of porcine NT eggs activated by a single electric stimulus were similar to those of parthenogenetic and *in vitro*-derived fertilization-derived (IVF) embryos. Thus, this invention demonstrates that a single electric stimulus is sufficient to mediate the development of porcine NT embryos using somatic cells.
Optimal activation time of reconstructed eggs with somatic cells after electrofusion is provided in this invention. In cattle, NT embryos activated at 4 to 8 h after fusion of donor cells with MII cytoplasts showed an improvement in embryonic development (Stice et al., Biol. Reprod., 54:100-110 (1996); Wells et al., Reprod. Fertil. Dev., 10:369-378 (1998)). A prolonged exposure of transferred nuclei to oocyte cytoplasmic factors also facilitated nuclear remodeling and reprogramming (DiBerardino et al., Science, 224:946-952 (1984); Ware et al., Gamete Res., 22:265-275 (1989)). Therefore, it suggests that allowing a lag time for the reconstructed oocytes to stand for a few hours prior to activation will lead to a better facilitation of the nuclear remodeling or reprogramming albeit the mechanism itself has not been clearly understood yet. To determine the conditions for an optimal activation of oocytes, the developmental abilities of porcine NT eggs induced by different activation times after electrofusion were examined. In the present invention, the porcine NT embryos activated 1 to 2 h after fusion showed an improved development as compared to those exposed to either shorter periods (0 and 0.5 h) or longer periods (4 and 6 h). This may be due to the difference in timing of genomic activation among different species. The activation of embryonic genome usually occurs at a 4-cell stage in pigs, whereas it occurs during an 8- to 16-cell stage in sheep and cattle (Kopscny, Reprod. Nutr. Dev., 29:589-600 (1989)). Thus, it is suggested that the activation time of reconstructed porcine oocytes after electrofusion will play a crucial role in producing viable embryos after somatic cell nuclear transfer. Further, it is also suggested that the time required for the remodeling or reprogramming of donor nuclei in recipient cytoplasts after fusion may differ from species to species.

Successful production of porcine cloned embryos from *in vitro* matured oocytes after nuclear transfer is also disclosed in the present invention. Most cloned animals such as sheep, goats, mice and pigs except cattle have been developed from NT embryos produced by transfer of nuclei of somatic cells into *in vivo*-derived oocytes after enucleation. *In vivo*-derived oocytes are obtained by flushing oviducts of animals superovulated by treating with gonadotropin hormones, which differ among species. However, the numbers of *in vivo*-derived oocytes are generally limited to a certain extent because the procedures are laborious and time-consuming. Alternatively, a lot of oocytes as recipient cytoplasts can be prepared *in vitro.* Immature oocytes of good quality are obtained from slaughterhouse-derived ovaries, matured *in vitro* and used as recipient cytoplasts after enucleation. Recently, there was a report that *in vivo*-derived oocytes could generate clone pigs(Onishi et al., Science, 289:1188-1190 (2000); Polejaeva et al., Nature, 407:505-509 (2000)). However, this invention provides a method for producing porcine NT embryos using *in vitro*-derived oocytes after somatic cell nuclear transfer.

It has been suggested that developmental ability of NT embryos with somatic cells may be different depending on cell types in mammals (Kato et al., Science, 282:2095-2098 (1999), Zakhartchenko et al., Mol. Reprod. Dev., 54:264-272 (1999)). Both cumulus and fetal fibroblast cells used as donor nuclei in the present invention supported the development of NT porcine embryos to the pre-implantation stage and this implies that the activation system of the present invention in nuclear transfer can employ various cell types.

In the present invention, NT embryos with transfected cells had similar developmental competence as compared to those with non-transfected donor cells. Moreover, transgenes were strongly expressed during early stage of development in all the NT embryos with transfected cells and this strengthens the possibility of generating transgenic embryos or transgenic animals from NT embryos with transfected cells after nuclear transfer.

### EXPERIMENTAL DETAILS

### In Vitro Maturation(IVM) and In Vitro Fertilization(IVF)

Prepubertal porcine ovaries were collected from a local slaughterhouse and transported to a laboratory and kept at 25-30°C in 0.9% (w/v) saline supplemented with 75 µg/ml potassium penicillin G and 50 µg/ml streptomycin sulphate until use. Cumulus-oocyte complexes (COC) were obtained from porcine follicles with a diameter of 3 to 6 mm using an 18-gauge needle connected to a 10 ml disposable syringe. The COC were washed 3 times with Tyrode's lactate (TL)-Hepes medium. Approximately 50 COC were cultured in each well of a 4-well multidish with 500 µl of maturation medium, respectively, (Nunc, Roskilde, Denmark) which were kept at 39°C, 5% CO₂ in air. After 20 to 22 h of culture, the oocytes were washed 3 times with TL-Hepes medium and further cultured in the maturation medium without supplement of hormones of pregnant mare's serum gonadotropin (PMSG) and human chorionic gonadotropin (hCG) for 22 h. The medium was covered with warm paraffin oil (light mineral oil) and equilibrated at 39°C, 5% CO₂ in air for at least 2 h prior to use. The medium used for oocyte maturation was bovine serum albumin (BSA) free North Carolina State University (NCSU) 23 medium supplemented with 10% (v/v) porcine follicular fluid, 0.57 mM cysteine, 25 µg/ml gentamycin, 10 ng/ml epidermal growth factor (EGF), 10 IU/ml PMSG and 10 IU/ml hCG. Porcine follicular fluid was collected from follicles of 3-6 mm in diameter, centrifuged at 1,600 x g for 30 min at 4°C, filtered through 1.2 µm syringe filters and stored in aliquots at -20°C until use.
The basic medium used for IVF was essentially the same as described by Abeydeera and Day (Biol. Reprod., 57:729-734 (1997)). This IVF medium, designated as modified Tris-buffered medium (mTBM), consists of 113.1 mM NaCl, 3 mM KCl, 7.5 mM CaCl₂ 2H2O, 20 mM Tris (crystallized free base; Fisher Scientific, Fair Lawn, NJ), 11 mM glucose, 5 mM sodium pyruvate and no antibiotics. Fresh porcine semen was kindly supplied from Darby Pig Al Center (Anseong, Korea) once a week and kept at 17°C for 5 days. The above semen was washed 3 times by centrifugation with Dulbecco's phosphate buffered saline (DPBS) supplemented with 1 mg/ml BSA (Fraction V), 100 µg/ml penicillin and 75 µg/ml streptomycin. At the end of the wash, the spermatozoa were resuspended in Tris-buffered medium (pH 7.8). After the completion of IVM, cumulus cells of oocytes were removed by treatment with 0.1% (w/v) hyaluronidase in NCSU 23 medium. The denuded oocytes were washed 3 times with the mTBM supplemented with 2.5 mM caffeine and 4 mg/ml BSA and placed into a 50 µl of mTBM under paraffin oil. Fifty microliter of diluted spermatozoa was added to 50 µl of fertilization medium containing oocytes to give a final sperm concentration of 5 × 10⁵ cells/ml. The oocytes were coincubated with spermatozoa for 6 h at 39°C in an atmosphere of 5% CO₂ in air.

### Preparation of Somatic cells

To prepare cumulus cells, COC were collected from a single porcine ovarv. After 46 h of culture for IVM, COC were treated in TL-HEPES medium supplemented with 0.1% (w/v) hyaluronidase to disperse cumulus cells. Cumulus cells were precipitated by centrifugation at 700 × g on a microcentrifuge (Vision Scientific Co., Korea) for 5min. The cell pellet was resuspended in Ca²⁺, Mg²⁺-free phosphate buffered saline (DPBS(-)), and the cell suspension was kept at room temperature for up to 2 h before use as donor nuclei. Fetal fibroblast cells were isolated from fetuses at day 40 of gestation in Korean native swine (*Sus scrofa domesticus* L.). Briefly, porcine fetuses were washed 3 times with DPBS(-). The heads of fetuses were removed using iris scissors, and soft tissues from liver and intestine were discarded by scooping out with two watch-maker's forceps. After washing twice with DPBS(-), the carcass was minced with a surgical blade on an 100 mm culture dish. The minced tissues were incubated in 10 ml of 0.25% (w/v) trypsin/3.65 mM EDTA solution at 39°C for 30 min. Trypsin activity was inhibited by adding an equal volume of cell culture medium supplemented with 15% fetal bovine serum (FBS; Gibco, Life Technologies Inc., Grand Island, NY). The cell culture medium was composed of Dulbecco's modified Eagle's medium (DMEM; Gibco), 15% FBS, 1,000 units of penicillin and 1,000 µg/ml of streptomycin (Gibco). After vigorous pipetting, the supernatant was centrifuged at 150 × g for 5 min. The cells were suspended, adjusted to a final concentration of 2 × 10⁶ cells /ml and then cultured in 10 ml of the culture medium in 175 cm² tissue culture flask (Nunc, Roskilde, Denmark) at 39°C, 5% CO₂ in air. The fetal fibroblast cells were passaged 3 times before using as a source of donor nuclei.

### Transfection of Foreign DNA into Somatic Cells

Polybrene/dimethyl sulfoxide (DMSO)-mediated transfection (Kawai and Nishizawa, Mol. Cell Biol., 4:1172-1174 (1984)) was performed as follows: 10 µg of plasmid pLNβ-eGFP DNA in 1 ml medium supplemented with 30 µg/ml polybrene (Aldrich, Milwaukee, WI, USA) was added to Gibbon Ape leukemia virus-based PG13 packing cells plated the previous day (5 X 10³ cells/60 mm dish). After 6 h of incubation at 37°C in an atmosphere of 5% CO₂, the DNA-medium mixture was aspirated, and packing cells were shocked by adding 2 ml of medium containing 25% DMSO and then incubated for 1 min. Following 3 washes with the medium, cells were fed with 5 ml of medium and incubated overnight before splitting the transfected cells. Infection of fetal fibroblast target cells was performed following the modified procedure of Miller and Rosman (Biotechniques, 7:980-982 (1989)); 3 ml of virus-containing medium filtered through a 0.22 µm pore-size filter, and polybrene (5 µg/ml of final concentration) was added to target cells which were plated the previous day. Exposure of the target cells to the mixture was allowed only 1 hr. The virus-containing medium was harvested from virus-producing cells, which had been fed with non-selection medium the previous day. Following 1 day of culture after infection, the infected cells were trypsinized and split in non-selection medium. In both transfection and infection, G418 selection medium (600 µg/ml) was added on the following day after splitting. The transfected cells were passaged 14 times before using as a source of nucleus donors.

### Enucleation of Porcine Oocytes

After 44 to 46 h of IVM, the oocytes were transferred to 500 µl of TL-Hepes medium supplemented with 0.1% hyaluronidase and free of cumulus cells by vortexing for 3 min. The zona pellucida of oocytes was partially dissected using a fine glass needle as described in Tsunoda et al. (J. Exp. Zool., 240:119-125 (1986)). Oocyte manipulation, including enucleation and cell injection, was performed by using a micromanipulator equipped with an inverted microscope (Leitz, Ernst Leitz Wetzlar GmbH, Germany). The medium used for oocyte manipulation was DPBS(-) containing 7.5 µg/ml cytochalasin B. The first polar body and partial cytoplasm presumptively containing metaphase 11 chromosomes were removed together by using a micropipette with an inner diameter of 20 µm. After the enucleation, oocytes were stained with 5 µM bisbenzimide (Hoechst 33342) for 5 min and observed under an epifluorescent microscope (Olympus, Tokyo, Japan).

### Cell Injection, Electrofusion and Activation

Single fibroblast cells were individually transferred into each enucleated oocyte. Small cells with smooth surface were used as a source of nucleus donors. Reconstructed embryos were equilibrated in a 50 µl drop of cell fusion medium for 10 to 20 sec, and transferred to a fusion chamber with two electrodes 1 mm apart overlaid with cell fusion medium. The cell fusion medium consisted of 0.3 M mannitol, 0.5 mM Hepes, 0.01% BSA, 0.1 mM CaCl₂ and 0.1 mM MgCl₂. Cell fusion was induced with a single DC pulse of 160 V/mm for 30 µsec using a BTX Electro-cell Manipulator 2001 (BTX, San Diego, CA, USA). After the electrofusion, embryos were activated by a 5 sec pulse of 3 V/mm AC followed by a 30 µsec pulse of 120 V/mm DC.

### Parthenogenetic Activation of Porcine Oocytes

Parthenogenetic activation of pig oocytes was carried out as described previously (Wang et al., Mol. Reprod. Dev., 51:346-353 (1998)). Briefly, cumulus-free oocytes were equilibrated for 1 min in 0.3 M mannitol solution supplemented with 0.5 mM Hepes, 0.01% BSA (fatty acid free), 0.01 mM CaCl₂ and 0.01 mM MgCl₂, and transferred to a chamber consisting of two electrodes 1 mm apart which was overlaid with activation solution. Oocytes were exposed to a single AC pulse of 3 V/mm for 5 µsec followed by a single DC pulse of 180 V/ mm for 30 µsec.

### In vitro Culture of NT Embryos

In all experiments, the activated eggs were cultured in 50 µl drops of NCSU 23 medium supplemented with 4 mg/ml BSA at 39°C, 5% CO₂ in air. After 72 h of culture, embryos at cleavage stage were selected. Forty to fifty cleaved embryos were cultured together in 50 µl drops of NCSU23 medium supplemented with 10% FBS at 39°C, 5% CO₂ in air for 3 days. After 6 days of culture, blastocyst formation was observed, and cell numbers of blastocysts were counted on a fluorescent microscope following Hoechst 33342 (2.5 µg/ml) staining.

### Cell Number of NT embryos

All blastocysts developed from NT embryos using somatic cells were counted for nuclei after Hoechst staining. Briefly, the embryos were fixed with 1% formaldehyde in PBS for 10 min at room temperature and then placed in a drop of mounting medium on a slide. The mounting medium consisted of 25% (v/v) glycerol in PBS containing 2.5 mg/ml sodium azide and 2.5 µg/ml Hoechst 33342. A cover slip was placed on the top of the embryos and the edge of the slide was sealed with fingernail polish. The nuclei numbers were counted on an epifluorescent microscope (Olympus, Japan).

This invention is explained in more detail based on the following Examples but they should not be construed as limiting the scope of this invention.

### EXAMPLE 1

It was examined whether different activation treatments after electrofusion affect *in vitro* development of NT porcine embryos to blastocyst stage. At 2 to 3 h after electrofusion, approximately one-third of reconstructed embryos were simultaneously allocated to 3 different activation groups; 1) embryos exposed to a 5 sec pulse at 3 V/mm AC followed by a 30 µsec pulse at 120 V/mm DC, 2) embryos activated by treatment with calcium ionophore (A23187, 5 µM for 5 min), and 3) embryos treated with A23187 (5 µM for 5 min) and then incubated in NCSU23 medium supplemented with 2 mM 6-DMAP for 4 h. After activation treatments, the reconstructed embryos from each group were washed 4 times and cultured in NCSU23 medium containing 4 mg/ml BSA. As shown in Table 1, differences were detected in the cleavage rate (58.1±13.9, 60.7±6.3 and 74.9±7.5%) and the developmental rate to blastocysts (2.2±2.8, 2.2±1.5 and 11.0±4.1%) among the groups (P<0.05). However, no significant difference in the mean cell number (20.5±3.5, 26.6±4.5 and 26.3±10.3) of blastocysts was observed. The results demonstrate that electric pulse could induce a higher *in vitro* development of NT embryos than treatments with A23187 alone or A23187/6-DMAP.

**Table 1.**

| **Effect of different activation treatments on *in vitro* development of NT porcine embryos with cumulus cells** | | | | |
|---|---|---|---|---|
| Treatment | No. of eggs examined | % of eggs cleaved | % of blastocysts | Cell number (Mean±SD) |
| A23187 | 128 | 58.1±13.9^{a} (71) | 2.2±2.8^{a} (2) | 20.5±3.5 |
| A23187+6-DMAP | 121 | 60.7±6.3^{a} (75) | 2.2±1.5^{a} (3) | 26.6±4.5 |
| Electric pulse | 129 | 74.9±7.5^{b} (95) | 11.0±4.1^{b} (13) | 26.3±10.3 |

| | | | | |
|---|---|---|---|---|
| These experiments were contemporaneously carried out 5 times for each group, respectively. ^{ab} Different superscripts within columns denote within significant difference (P<0.05). The number of embryos examined is indicated in parentheses. | | | | |

### EXAMPLE 2

The effect of activation voltage on *in vitro* development of NT embryos to the blastocyst stage was examined. At 2-3 h after electrofusion, the reconstructed embryos were activated by a pulse of 3 V/mm AC for 5 sec followed by a pulse of 120 or 150 V/mm DC for 30 µsec. There was no significant difference in the cleavage rate between the two groups. The rate of blastocyst development (11.6±1.6%, 14/121) of NT embryos in field strength of 120 V/mm was significantly high as compared to that of 150 V/mm group (6.5±2.3%, 8/121) (P<0.05). However, mean cell numbers of blastocysts were similar between the two groups (29.5±11.3 and 27.6±10.5, respectively) (P>0.05). For subsequent experiments, the activation was performed with a single 120 V/mm DC pulse (Table 2). The experiment was carried out to determine the optimal activation timing after electrofusion (Table 3). It was also investigated if the timing of activation after electrofusion affects the developmental ability of NT embryos. Approximately one-third of fused eggs were activated by a pulse of 3 V/mm AC for 5 sec followed by a single pulse of 120 V/mm DC for 30 µsec on different time (2, 4 or 6 h) after electrofusion. The cleavage rate of NT embryos in 2 h and 4 h groups was higher than that of 6 h group. A higher proportion of NT embryos (11.6±2.9%, 20/187) activated 2 h after electrofusion developed to the blastocyst stage, as compared to those of 4 h (6.6±2.3%, 11/190) and 6 h (8.1±3.3%, 14/184) groups. Mean cell numbers of NT embryos in 2, 4 and 6 h groups were 30.4±10.4, 24.6±10.1 and 16.5±7.4, respectively. A significant difference was observed between 2 h and 6 h groups (P<(1.05). When reconstructed embryos were activated at 0, 30 min and 1 h after electrofusion, in subsequent experiments, developmental rates of the embryos blastocyst stage were 2.1% (2/97), 3.2% (2/85) and 10.4% (11/106), respectively. These results suggest that activation timing after electrofusion can affect developmental potential of porcine NT eggs.

**Table 2.**

| **Effect of activation voltage on *in vitro* development of porcine NT embryos with somatic cells** | | | | |
|---|---|---|---|---|
| Group (V/mm DC) | No. of eggs examined | %* of eggs cleaved | %** of blastocysts | No. of Nuclei (Mean±SD) |
| 120 | 121 | 71.4±10.9 (86) | 11.6±1.6^{a} (14) | 29.5±11.3 |
| 150 | 121 | 63.7±8.1 (78) | 6.5±2.3^{b} (8) | 27.6±10.5 |

| | | | | |
|---|---|---|---|---|
| %*: No. of eggs cleaved/No. of eggs examined ×100. | | | | |
| %**: No. of blastocysts/No. of eggs examined ×100. | | | | |
| ^{ab} Different superscripts within column denote a significant difference (P<0.05). The number of embryos examined is indicated in parentheses. | | | | |

**Table 3.**

| ***In vitro* development of porcine NT embryos activated at different times after electrofusion** | | | | |
|---|---|---|---|---|
| Activation time | No. of eggs examined | % of eggs cleaved | % of blastocysts | No. of Nuclei (Mean±5D) |
| 2 h | 187 | 71.8±13.2^{a} (140) | 21.6±2.9^{a} (20) | 30.4±10.4^{a} |
| 4 h | 190 | 76.5±6.8^{a} (140) | 6.6±23^{b} (11) | 24.6±10.1^{ab} |
| 6 h | 184 | 59.4±4.9^{b} (110) | 8.1±3.3^{ab} (14) | 16.5±7.4^{b} |

| | | | | |
|---|---|---|---|---|
| ^{ab} Different superscripts within column denote a significant difference (P<0.05). The number of embryos examined is indicated in parentheses. | | | | |

### EXAMPLE 3

The developmental competence of NT embryos with fetal porcine fibroblast cells was compared to that of parthenogenetic and IVF-derived porcine embryos. And also, developmental potential of NT porcine embryos with cumulus or fibroblast cells was compared to that of IVF-derived embryos.

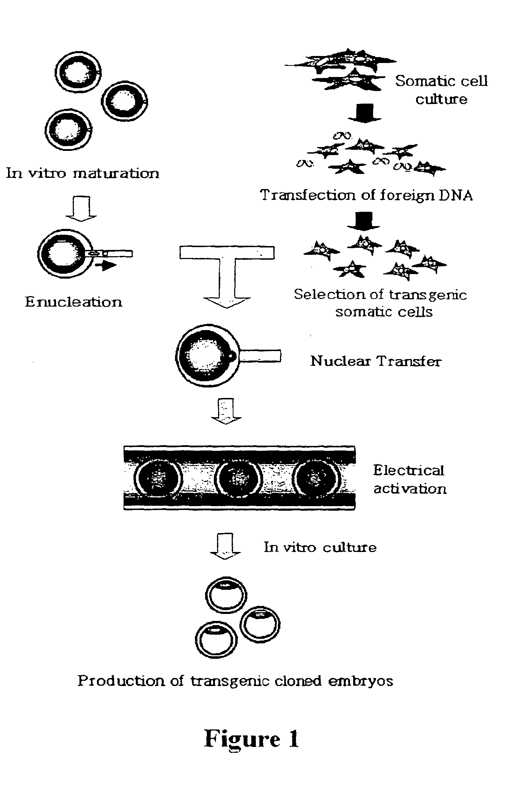

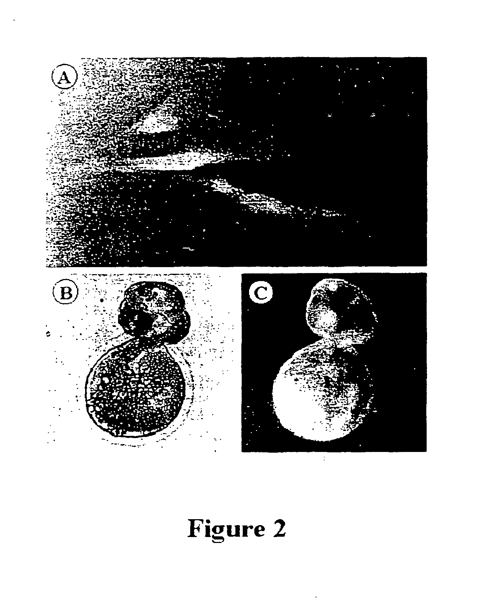

NT embryos using fibroblast cells showed a lower developmental rate (9.4±(0.9%, 20/289) to the blastocyst stage than IVF-derived (21.4±1.9%, 43/201) or parthenogenetic embryos (22.4±7.2%, 43/189) (P<0.01), although there was no significant difference in the cleavage rate of the embryos among experimental groups (Table 4). After 6 days of culture, most blastocysts (16/20) from NT embryo were hatched (Figure 1), although the mean number of nuclei was smaller than that of IVF-derived blastocysts (Figure 2). The reason for the early hatching of NT blastocysts seems to be due to the partial dissection of zona pellucida during the enucleation process. The mean cell number of NT embryos (28.9±11.5, ranging from 14 to 61, n=18) was smaller than that of IVF-derived blastocysts (36.2±9.7, ranging from 18 to 67, n=22) (P<0.05), while it was similar to that of parthenogenetic embryos (29.9±12.1, ranging from 13 to 50, n=24) (Table 4). These results represent that the porcine NT embryos using fibroblast cells could develop to blastocysts *in vitro*, although they had a lower developmental competence when compared to that of IVF-derived embryos.

Developmental potential of porcine oocytes, where nuclear transfer was performed using cumulus (NT^{†}) or/and fibroblast cells (NT^{‡} ), was compared to that of IVF-derived embryos (Table 5). Fusion rates of enucleated oocytes reconstructed with cumulus or fibroblast cells after electrofusion were 67% (257/374) or 69% (273/396), respectively. Thus, there was no distinct difference in the cleavage rate of NT embryos between 2 donor cell types. NT embryos from both cell types, however, showed a lower developmental rate to the blastocyst stage than that of IVF-derived embryos (P<0.01). Mean nuclei numbers of IVF-derived, NT^{†} and NT^{‡} blastocysts were 38.6±10.4 (ranging from 23 to 65), 28.9±11.4 (ranging from 17 to 51) and 30.2±9.9 (ranging from 18 to 51), respectively. Thus, NT embryos were comprised of number of nuclei smaller than those of IVF-derived embryos (P<0.05).

**Table 4.**

| **Comparison of *in vitro* development of NT porcine embryos to IVF-derived or parthenogenetic embryos** | | | | |
|---|---|---|---|---|
| Group | No. of eggs examined | % of eggs cleaved | % of blastocysts | Nuclei no. (Mean±SD) |
| IVF | 201 | 74.5±1.7 (150) | 21.4±1.9^{a} (43) | 36.2±9.7^{a} (22) |
| Parthenotes | 189 | 71.0±4.9 (133) | 22.4±7.2^{a} (43) | 29.9±12.1^{b} (24) |
| NT | 212 | 67.0±7.6 (141) | 9.4±0.9^{a} (20) | 28.9±11.5^{b} (18) |

| | | | | |
|---|---|---|---|---|
| ^{ab} Different superscripts within column denote a significant difference, P<0.01 for blastocysts, P<0.05 for nuclei number. The number of embryos examined is indicated in parentheses. | | | | |

**Table 5.**

| Comparison of *in vitro* development of IVF or NT porcine embryos with cumulus cells | | | | |
|---|---|---|---|---|
| Group | No. of eggs examined* | % of eggs cleaved | % of blastocysts | Cell number (Mean±SD) |
| IVF | 270 | 78.0±3.6 (210) | 22.9±3.5^{a} (62) | 38.6±10.4^{a} |
| NT^{†} | 257 | 70.6±9.9 (188) | 9.9±2.4^{b} (26) | 28.9±11.4^{b} |
| NT^{‡} | 273 | 73.8±5.3 (202) | 9.8±1.6^{b} (27) | 30.2±9.9^{b} |

| | | | | |
|---|---|---|---|---|
| * Six experiments were contemporaneously carried out for each group, respectively. | | | | |
| † NT porcine embryos with cumulus cells; | | | | |
| ^{‡} NT porcine embryos with fibroblast cells. | | | | |
| ^{ab} Values with different superscripts are different significantly, P<0.01 for blastocysts, P<0.05 for cell number. The number of embryos observed is indicated in parentheses. | | | | |

### EXAMPLE 4

It was examined whether porcine NT embryos with transfected cells develop to blastocysts and express the transgenes during early development. Fibroblasts expressing GFP gene were transferred into enucleated oocytes and the reconstructed oocytes were fused by a single DC pulse of 160 V/mm for 30 µsec. The fused embryos were activated and then cultured as described in EXAMPLE 2. GFP expression in NT embryos was observed under an epifluorescence microscopy (Olympus). Developmental potential of porcine NT oocytes with transfected cells was compared with that of non-transfected cells.

It was also examined whether transfected cells have *in vitro* developmental potential following nuclear transfer in the pig. Porcine fibroblast cells were introduced with exogenous β-actin promoter/GFP gene by retroviral infection, and surviving colonies were obtained after treatments of G418 for approximately 2 weeks. GFP expression in transfected fibroblast cells was confirmed by epifluorescence microscopy (Figure 1A). Fusion rate (67%, 195/292) of NT oocytes with transfected fibroblast cells was similar to that (71 %, 197/277) of non-transfected cells. Developmental rates of NT embryos to blastocvsts were also similar between transfected and non-transfected cells (Table 6). All NT embryos derived from transfected cells showed strong GFP expression as represented in Figure 1C. The results indicate that porcine NT embryos with transfected cells have a similar developmental potential like those with non-transfected cells. Thus, our invention methods for activating NT embryos are applicable to generate transgenic NT embryos from transfected cells.

**Table 6.**

| ***In vitro* development of porcine NT embryos with transfected cells** | | | | |
|---|---|---|---|---|
| Group | No. of eggs fused* | % of eggs cleaved | % of blastocysts | Expression rate (%) |
| Normal | 197 | 73.0±4.2 (144) | 10.2±0.6 (20) | - |
| Transfected | 195 | 75.7±:1.8 (148) | 9.7±0.5 (19) | 100 |

| | | | | |
|---|---|---|---|---|
| * Four experiments were contemporaneously carried out for each group, respectively. | | | | |

The number of embryos observed is indicated in parentheses.

## Claims

1. A method for mass production of porcine clone embryos by nuclear transfer comprising:
(a) a process of reconstructing enucleated porcine oocytes which are fused with porcine donor somatic cells or porcine donor somatic cell nuclei injected into said enucleated oocytes by electric stimulation; wherein the period of reconstruction is for 1-2 hours;
(b) a process of activating said reconstructed porcine oocytes by secondary electric stimulation by applying a single electric pulse of 120-150V/mm DV.

2. The method for mass production of porcine clone embryos by nuclear transfer according to claim 1, wherein said porcine donor somatic cell or said porcine donor somatic cell nucleus injected into said enucleated oocyte is a transfected somatic cell or a transfected somatic cell nucleus obtained from a group consisting of fibroblast cells and cumulus cells of mammals excluding human.

3. The method for mass production of porcine clone embryos by nuclear transfer according to claim 1, wherein said porcine oocytes as recipient cytoplasts are matured *in vitro* prior to enucleation.

4. The method for mass production of porcine clone embryos by nuclear transfer according to claims 1∼2, wherein said donor cells or donor cell nuclei are obtained from fetuses or adult tissues.

5. The method for mass production of porcine clone embryos by nuclear transfer according to claim 1, wherein said secondary electric stimulation is to apply a single electric pulse of 120∼150 V/mm DC for 30 µsec.

6. The method for mass production of porcine clone embryos by nuclear transfer according to claims 1∼2 and 4, wherein said donor somatic cells or donor somatic cell nuclei in pigs are obtained from a swine indigenous to Korea (Sus *scrofn domesticus* L.).

## Patentansprüche

1. Verfahren zur Massenproduktion von Schweineklon-Embryos durch Kerntransfer, das folgendes umfasst:
a) ein Verfahren zum Wiederherstellen entkernter Schweine-Eizellen, die mit somatischen Spenderzellen oder somatischen Spenderzellkernen vom Schwein, die in die entkernten Eizellen durch elektrische Stimulierung injiziert werden, fusioniert werden, wobei die Zeitspanne der Wiederherstellung 1 bis 2 Stunden beträgt;
b) ein Verfahren zum Aktivieren der wiederhergestellten Schweine-Eizellen durch eine sekundäre elektrische Stimulierung durch Anlegen eines einzigen elektrischen Impulses von 120-150 V/mm DC.

2. Verfahren zur Massenproduktion von Schweineklon-Embryos durch Kemtransfer gemäß Anspruch 1, wobei die somatische Spenderzelle oder der somatische Spenderzellkern vom Schwein, die in die entkernte Eizelle injiziert werden, eine transfizierte somatische Zelle oder ein transfizierter somatischer Zellkern ist, gewonnen aus einer Gruppe, die aus Fibroblastenzellen und Kumuluszellen von Säugetieren, ausgenommen dem Menschen, besteht.

3. Verfahren zur Massenproduktion von Schweineklon-Embryos durch Kemtransfer gemäß Anspruch 1, wobei die Schweine-Eizellen als Empfängerzytoplasten in vitro vor der Entkemung gereift werden.

4. Verfahren zur Massenproduktion von Schweineklon-Embryos durch Kemtransfer nach Anspruch 1 - 2, wobei die Spenderzellen oder Spenderzellkerne von Föten oder adulten Geweben gewonnen werden.

5. Verfahren zur Massenproduktion von Schweineklon-Embryos durch Kemtransfer nach Anspruch 1, wobei die sekundäre elektrische Stimulierung darin besteht, einen einzigen elektrischen Impuls von 120 - 150 V/mm DC für 30 µs anzulegen.

6. Verfahren zur Massenproduktion von Schweineklon-Embryos durch Kemtransfer gemäß Anspruch 1 - 2 und 4, wobei die somatischen Spenderzellen oder die somatischen Spenderzellkerne in Schweinen aus einem Schwein gewonnen werden, das in Korea beheimatet ist (Sus scrofa domesticus L.)

## Revendications

1. Procédé pour la production en masse d'embryons de clone porcin par transfert nucléaire comprenant :
(a) un processus de reconstruction des ovocytes porcins énucléés qui sont fusionnés avec des cellules somatiques de donneur porcin ou des noyaux de cellules somatiques de donneur porcin injectés dans lesdits ovocytes énucléés par stimulation électrique, dans lequel la période de reconstruction est de 1 à 2 heures.
(b) un processus d'activation desdits ovocytes porcins reconstruits par seconde stimulation électrique secondaire en appliquant une impulsion électrique unique de 120-150 V/mm CC.

2. Procédé pour la production en masse d'embryons de clone porcin par transfert nucléaire selon la revendication 1, où ladite cellule somatique du donneur porcin ou ledit noyau de cellule somatique du donneur porcin injecté dans ledit ovocyte énucléé est une cellule somatique transfectée ou un noyau de cellule somatique transfectée obtenue d'un groupe composé de (cellules) fibroblastes et cellules de cumulus de mammifères à l'exception des humains.

3. Procédé pour la production en masse d'embryons de clone porcin par transfert nucléaire selon la revendication 1, où lesdits ovocytes porcins en tant que cytoplastes receveurs sont maturés *in vitro* avant énucléation.

4. Procédé pour la production en masse d'embryons de clone porcin par transfert nucléaire selon les revendications 1 à 2, où lesdites cellules donneuses ou lesdits noyaux de cellules donneurs sont obtenus à partir de foetus ou de tissus adultes.

5. Procédé pour la production en masse d'embryons de clone porcin par transfert nucléaire selon la revendication 1, ou ladite seconde stimulation électrique secondaire a pour but d'appliquer une impulsion électrique unique de 120-150 V/mm CC pendant 30 µsec.

6. Procédé pour la production en masse d'embryons de clone porcin par transfert nucléaire selon les revendications 1 à 2 et 4, où lesdites cellules somatiques donneuses ou lesdits noyaux de cellules somatiques donneurs de porcs sont obtenus d'un porc (ou suidé) indigène de Corée *(Sus scrofa domesticus L.).*
